Europäisches Patentamt

European Patent Office

Office européen des brevets

(11) Publication number: **0 094 613**
**B1**

(12) **EUROPEAN PATENT SPECIFICATION**

(45) Date of publication of patent specification: **10.12.86**

(21) Application number: **83104647.9**

(22) Date of filing: **11.05.83**

(51) Int. Cl.⁴: **A 61 F 5/448**

(54) **Apparatus for tending a stoma.**

(30) Priority: **13.05.82 DE 3218092**

(43) Date of publication of application:
**23.11.83 Bulletin 83/47**

(45) Publication of the grant of the patent:
**10.12.86 Bulletin 86/50**

(84) Designated Contracting States:
**AT BE CH DE FR GB IT LI LU NL SE**

(56) References cited:
**DE-A-1 616 476**
**DE-U-7 602 839**
**DE-U-7 811 660**
**FR-A-1 232 413**
**GB-A- 839 818**
**GB-A-1 568 860**
**GB-A-1 578 020**
**US-A-3 439 679**

(73) Proprietor: **E.R. Squibb & Sons, Inc.**
**Lawrenceville-Princeton Road**
**Princeton, N.J. 08540 (US)**

(72) Inventor: **Hunger, Gerd, Dr. Med.**
**Höslstrasse 8**
**D-8000 Munich 81 (DE)**

(74) Representative: **Vossius & Partner**
**Siebertstrasse 4 P.O. Box 86 07 67**
**D-8000 München 86 (DE)**

Courier Press, Leamington Spa, England.

# Description

The tending of a stoma — i.e. of an artificial outlet for stool (ileostoma, colostoma) or urine (urostoma) — is effected nowadays mainly by means of disposable adhesive bags.

Apart from the (rare) allergy to the adhesive, the adhesive bags involve the disadvantage that when being replaced they tear off or "strip" the uppermost epithelium layers of the skin until it wets and becomes extremely susceptible to irritations and infections. Skin problems around the stomata render the application of adhesive bags impossible in many cases, above all when the stripped skin is additionally exposed to fermentatively aggressive excreta (ileostomy, urostomy) or infectious material (fistulae).

To protect the skin in the immediate vicinity of the stoma, there have been developed base plates of a hypoallergic material, comprising a thin elastic film on the outside of the base plate. The base plates may remain in the applied position for several days. Onto this artificial skin the bags are adhered and are also to be replaced there once or several times a day.

This solution is extremely convenient to the patient (all is flat and flexible) and theoretically represents actually the ideal solution. In practical use, however, there result serious disadvantages. During sitting and stooping, folds are forming in the base plate so that, when the bag is being replaced, an odor and liquid-tight closure is made impossible. Moreover, the covering film of the base plate, which is very thin so as to have as far as possible the elasticity of the skin, is often damaged upon stripping off a bag. The base plate thereby becomes unserviceable and must prematurely be replaced.

To overcome these said disadvantages, it has therefore been suggested to glue directly onto the base plate the semi-rigid ring of a snap closure whose likewise semi-rigid counterpart is provided at the bag. The closure engages according to the snap-fastener principle and can be replaced. This system solves very well the replacement of the bags but involves other, very inconvenient disadvantages:

1. The closure becomes practically rigid by the junction of the two semi-rigid rings. This rigid closure is very uncomfortable during sitting and stooping — quite particularly when large-size stomata, requiring large-size closures, are involved. The closure exerts a painful pressure on the skin, it detaches, due to its rigidity, the base plate from the elastic skin and finally "uses up" the base plate so that it has to be replaced more often than would be necessary with a fully elastic closure.

2. On account of the inconveniencies during wearing, the patient cannot be expected to put up with a ring larger than absolutely necessary. There is thus required a considerable number of ring sizes both for the base plate and for the bag (at present 5 sizes are commercially available). Production and distribution are thereby rendered more expensive.

3. The tendance of the most critical place, namely the skin ring between the circumference of the stoma and the inner edge of the circular opening of the base plate — e.g. by means of a coating paste — is complicated if narrow snap rings are involved. Wide rings allow a good tendance of that critical skin ring but are bulky and very uncomfortable due to their rigidity.

4. Moreover, excreta are regularly gathering in the circular niche between base plate and snap ring. They can be removed only with difficulty, decompose quickly and lead to additional undesirable odors. They render a clean replacement difficult or impossible.

5. When the bag is being replaced, the new bag must be connected to the ring on the side of the body by pressure. Since said ring is firmly attached to the base plate, it is not possible to exert a counterpressure with the fingers. The pressure necessary for effecting the connection therefore fully acts on the at first mostly very sensitive abdomen of the patient.

The original and correct concept of a flexible connection between base plate and bag has, on account of the difficulties in the bag replacement, been sacrificed thus in favor of a rigid connection, which admittedly enables a simple replacement but involves said disadvantages.

With the particular object to overcome the disadvantages stated above under 1. and 5., there has already been proposed a device (Product Information "Two-Piece Ostomy System", Hollister Inc., March 1982), in which the ring of the snap closure is not glued to the base plate but connected thereto through a connecting piece in such a way that a certain movability of the two parts relative to each other is provided. The connecting piece is a flat (non-cylindrical or non-conical) disk of a plastic foil, connected at its outer circumference to the external portion of the back of the snap ring. The connection to the base plate is provided in the central area of the disk around the central opening.

The connection between base plate and snap ring is in this known device still relatively rigid. In the rest position, the snap ring substantially lies against the base plate — an all-round, even small space between base plate and ring is not provided in that arrangement.

Consequently, the pressure discomfort to the user during stooping and sitting remains substantially unchanged. Since the connecting piece is fastened in its central portion around the central opening at the base plate, the possibility for the user to enlarge the opening so as to adjust it to his individual requirements is very restricted. In this device, too, various sizes are thus necessary, which renders the production, distribution and use more complicated and expensive.

Moreover, it follows from the use of a flat connecting piece and the central adhesion bonding necessary for the desired "floating" of the ring that a considerable distance must exist between the inner adhesion point and the ring. Therefore, a disproportionately large and thus uncomfortable ring is required for a given stoma size.

Finally, the snap ring of that device comprises on its inner side the necessary undercut for holding the counterpiece, which represents an undesired soil-catcher. Soil is collecting furthermore between the inner region of the bottom side of the ring and the connecting piece.

As advantage of the described known device there remains thus substantially only the fact that it is possible to tilt the ring so as to enable to put the fingers below the snap ring and to exert a counterpressure when pressing on the ring provided at the bag. However, due to the close fitting of the snap ring at the base plate, a pressure molestation of the area surrounding the stoma cannot be completely avoided in that device either. As regards the movability of the parts relative to each other, the desired reduction of the number of sizes of the product and the demands concerning the requirements for cleaning, much is still left, however, to be desired.

GB—A—1 578 020 discloses an ostomy device comprising a bushing having a large second flange secured to the front face of a self-adhesive PVC skin and a smaller resilient first flange. To the first flange an ostomy container is secured either before or after application to the user's skin. The bag must be held in place in a flange using a rubber band.

FR—A—1 232 413 describes a collecting bag with a base plate and a rigid tapered neck ending in an annular bead. The upper part of the neck comprises a hook and the lower part comprises a projection. The neck forms a basis for the elastic ring of the bag, and the neck is rigid in order not to be compressed by the elastic ring of the bag, whereby the junction between neck and bag would loose its tightness and would leak.

DE—A—1 616 476 discloses a closure comprising a rigid ring being sealingly fixed to a base plate by means of a flange. When applying the bag, the rigidness of the ring is enhanced by another ring also consisting of a hard plastic material.

The invention solves the problem of providing a device for tending a stoma, by providing a base plate and a ring connected therewith through a connecting piece, and a bag for taking up the excreta being attached to said ring. This device is to enable a permanent elasticity and flexibility after connection of base plate and bag and simultaneously a trouble free replacement of the bag.

This problem is solved according to the invention by a device of the stated kind, in which the connecting piece between ring and base plate is a flexible cylindrical or conical connecting piece.

Subject matter of the invention is thus a device for tending a stoma comprising an apertured base plate for securing the device to the skin surrounding the stoma, a ring connected with said base plate via a flexible connecting piece, and a bag for taking up the excreta and being detachably attached to the ring, characterized in that the connecting piece between the ring and the base plate

(a) is cylindrical or conical,

(b) extends perpendicularly to the base plate or obliquely downwards, and

(c) has a diameter which compared with the base plate is as large as possible.

Brief description of the drawings

Figure 1 shows an embodiment of the invention consisting of base plate, flexible cylindrical or conical connecting piece welded or glued onto it and flexible but selfsustaining flat ring for adhering the bag. The counterpart thereto is the likewise shown adhesive ring of the bag which comprises a somewhat larger inner diameter than the ring for adhering the bag connected to the base plate. (For reasons of clearness, the parts are shown in exploded view in Fig. 1);

Figure 2 shows a section through the embodiment according to Fig. 1 parallel to the axis of the cylindrical or conical connecting piece;

Figure 3 shows an embodiment of the invention in which the cylindrical or conical connecting piece is extended beyond the ring for adhering the bag. In this case the shape of the connecting piece can be changed after passage through the ring;

Figure 4 shows an embodiment of the invention in which the cylindrical or conical connecting piece is likewise extended beyond the adhesive ring and terminates in a nonreturn valve.

Fig. 4a: section parallel to the axis of the cylindrical or conical connecting piece;

Fig. 4b: topview in the direction of the base plate (adhesive ring not shown).

Figure 5 shows embodiments of the device in which the ring is formed as snap ring of a conventional snap closure.

Fig. 5a: illustrates a known prior art embodiment wherein a base plate and snap ring are directly attached, producing a sharp annular edge. The undercut on the inner side of the ring forms a soil catcher;

Fig. 5b; illustrates an alternative embodiment according to the invention. The circular edge between base plate and cylindrical or conical connecting piece is rounded off. The connecting piece engages at the inner circumference of the snap ring. Thereby the formation of soil-catching edges and niches is prevented. The cylindrical or conical connecting piece is extended beyond the snap ring — optionally by changing its geometric shape — and thus acts as a soil repeller. The undercut is situated on the outside of the ring.

The connecting plate (2) between base plate (1) and ring (3), which constitutes an essential element of the invention, can be cylindrical or conical. It consists preferably of a flexible plastic foil. The connecting piece has a diameter which compared with the base plate is as large as possible, inter alia to ensure an optimum distribution of the tensile load by the filled bag (4). Thereby the danger of a separation of the base plate from the skin is largely excluded, which would happen if the tension is concentrated on a small area. The cylindrical or conical connecting piece has a height of at least 1 mm. A height of 3 to 15 mm is preferred.

The connection of the flexible connecting piece to the base plate is effected preferably on an annular surface of the base plate which has a diameter only insignificantly smaller than that of the ring. Accordingly, the connecting piece has a cylindrical or slightly conical form. Thus, an optimum utilization of the size of ring and base plate is achieved since the user can, depending on his individual requirements, widely enlarge the central openings of the base plate and is not confined in this connection by a central adhesion area between base plate and connecting piece. Apart from a cylindrical embodiment of the connecting piece, however, also a (slightly) conical design is possible and subject matter of the invention. That design is advantageous as regards the discharge from the stoma into the bag but comparatively disadvantageous for other reasons already stated, above all when the cone comprises a greater taper in the direction of the base plate.

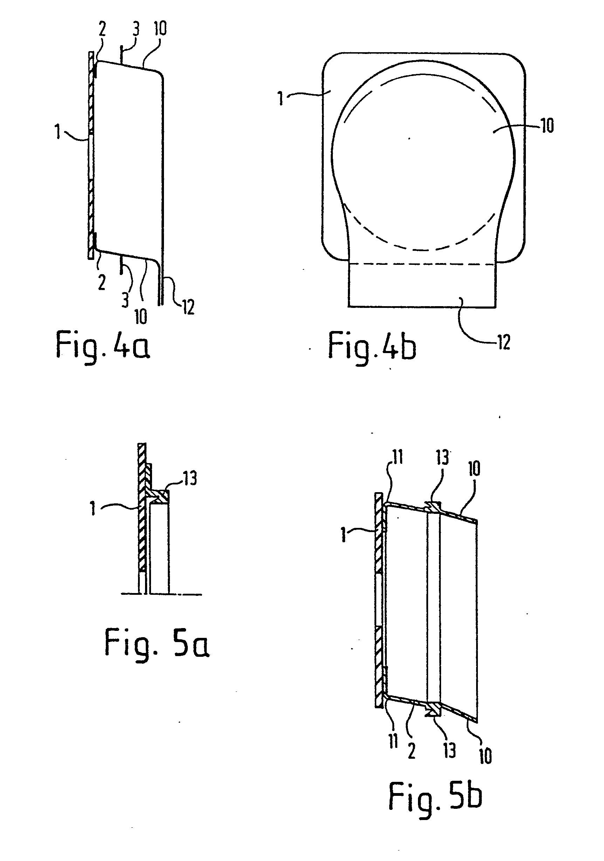

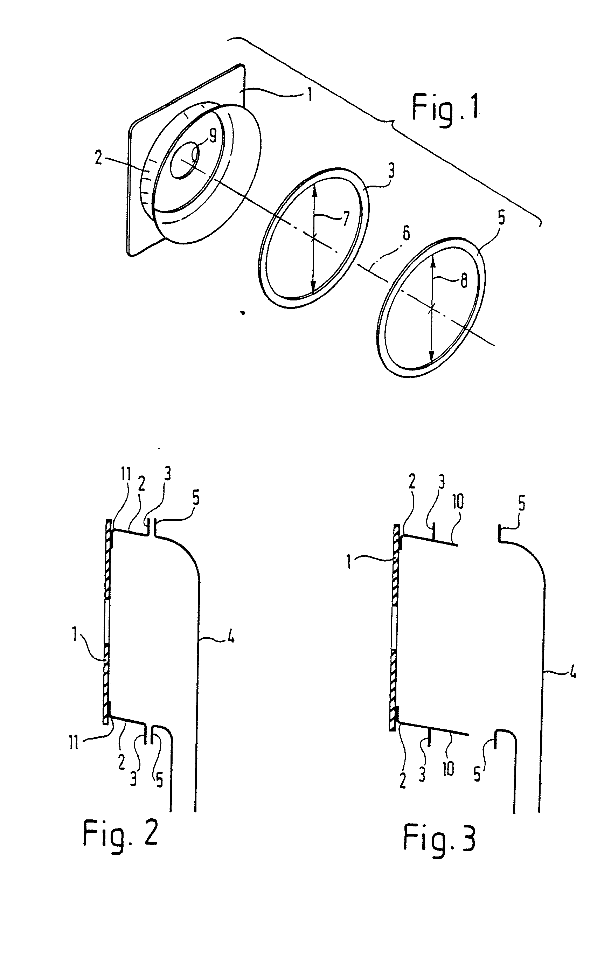

The axis 6 of the flexible cylindrical or conical connecting piece (hereinafter designated "connecting piece" for the sake of brevity) can be perpendicular to the base plate, or it may be advantageous to direct it obliquely downwards so that the formation of folds is minimal and excreta can flow off spontaneously. For the same reason it is preferred that the edge 11 between base plate and connecting piece is rounded off; See Figure 2.

In a preferred embodiment of the invention a flat, flexible but selfsustaining ring 3 for adhering the bag 4 is connected via the flexible connecting piece before the base plate, to which ring the conventional bags 4 with adhesive ring 5 can be attached and replaced without difficulty. The ring for adhering the bag is preferably disposed parallel to the plane of the base plate and is — connected by the connecting piece — spaced only some mm therefrom. The ring for adhering the bag has preferably a width of from 0.5 to 1 cm and simultaneously is as thin and flexible as possible but selfsustaining. Any normal adhesive bag can be fastened to it and removed therefrom for replacement. In contrast to the base plate, which — while being almost as elastic as the skin — gets grooves and furrows, i.e. is deformable, the adhesive ring is flexible but non-deformable. These two properties, which hitherto could not be combined in a base plate, are realized by the invention in different parts of the device. The adhesive bags will sealingly engage the non-deformable ring for adhering the bag even after many replacements have been effected, whereas a direct sealing on the base plate becomes problematic already after a short time of wearing.

In the device of the invention the conventional base plates can be used, which consist preferably of a hypoallergic material having a thin elastic film on the outside. However, there may also be used as "base plates" simple films with the adhesives customary in connection with adhesive bags. Since the base plate has to be replaced only at intervals of several days, the skin damaging by stripping is minimal also in this simple and economic embodiment, in contrast to adhesive bags which have to be replaced once or several times a day on the skin.

The manner of connecting the connecting piece to the base plate is not critical; due to the easy performance, a welding or gluing is preferred in practice. The edge between the base plate and the connecting piece is preferably rounded off so as to keep a soiling by excreta as low as possible.

The invention comprises the following advantages:

1. The device is fully elastic, and even after fastening the bag is comfortable — whereas a snap closure consisting of two semi-rigid parts is practically rigid after engagement of the bag, and is uncomfortable.

2. The device preserves the base plate, which — in contrast to the mechanical strain of a rigid ring — does not cant with the ring during sitting and stooping nor is it physically displaced. It can be worn for a longer period (economic advantage) and has to be replaced less frequently (convenience for the patient).

3. The device is cleaner since in the circular grooves and niches of the snap closure remainders of excreta will always be caught and can be removed only with difficulty, whereas in this device such soil catchers do not exist. This applies particularly if the inner diameter 8 of the bag opening is about 2 to 3 mm greater than the inner diameter 7 of the ring for adhering the bag.

4. The device is superior to the conventional devices as regards the tending of the most critical area, namely the immediate circumference of the stoma. That area must often be provided with pastes. In the device according to the invention the access to that area is optimal, since the connecting piece is as wide as the size of the base plate permits and since the flat ring for adhering the bag, in contrast to the snap closure, is not bulky.

5. The device is cheaper than a snap closure since the thin adhesion closure requires substantially less material than the robust snap closure. The device is moreover cheaper since only one size or at least only a small number of sizes are required. Large rings for adhering the bags are not less comfortable than small ones. The manufacture of a number of different sizes of the device is unnecessary. As to the uncomfortable snap closure, on the other hand, no patient can be expected to wear a larger closure than absolutely necessary. Therefore numerous sizes thereof are commercially available.

6. Since the connecting piece is connected to the base plate in the outer area thereof so as to be more or less coincident with the ring, the user of the device according to the invention can enlarge the opening for the stoma in the base plate within relatively wide limits, in accordance with his individual needs. In particular with respect to the large stomata the tending of which is most difficult, there is achieved thereby a more advantageous ratio between the size of the stoma and that of the ring than would be possible with a connection between base plate and connecting piece situated in the central area of the base plate.

7. The connection between the ring for adhering the bag and adhesive bag can be effected without any pressure on the skin surrounding the stoma which is quite sensitive. In this respect, too, a quite considerable advance is achieved in comparison with the known arrangements.

8. The danger of a spontaneous snapping open of the closure, a serious problem with large rings e.g. upon an intense stooping, is practically eliminated by the adhesion connection and on account of the extensive movability of the closure relative to the base plate, respectively.

According to a further improvement, the device of the invention is provided with a soil repeller 10. For this purpose the connecting piece is wholly or partially extended beyond the elastic ring for adhering the bag (see Figure 3), with the possibility of being narrowed e.g. to a truncated cone. The ring for adhering the bag then will not be soiled when the bag is being replaced. A further improvement in this respect is provided by the feature that the film on the base plate as well as the inner side of the movable connecting piece is siliconized so as to achieve a complete discharge into the bag. The soil repeller can also be extended to a nonreturn valve 12; see Figure 4. Then the sensitive skin in the direct surroundings of the stoma is protected primarily at night against a flowing back of the aggressive or infectious excreta.

Devices comprising a snap closure involve the described disadvantages and are thus actually not preferred in the present invention. Apart from the disadvantages of the snap closure, the latter comprises, on the other hand, certain advantages, particularly a trouble free and tight closing. Therefore, according to a further embodiment of the invention, devices with snap closures can be improved by the insertion of the movable cylindrical or conical connecting piece between base plate and the body-facing ring of the snap closure; see Figure 5. The undercut of the body-facing snap ring 13 is provided in this embodiment preferably at the outer circumference of the ring. With this arrangement the following advantages are achieved:

1. The flexible connecting piece reduces the discomfort to the patient by the rigid closure, mainly if with the rigid closure it is sufficiently long and thus provides sufficient clearance;

2. The connecting piece eliminates soil-catching niches if it seals the inner side of the closure ring facing the base plate;

3. A further improvement is possible if the flexible connecting piece is, in accordance with Figure 3, extended as a soil repeller and thus enables a cleaner replacement of the bags. Moreover, there is provided the possibility of extending the connecting piece to a nonreturn valve.

## Claims

1. Device for tending a stoma comprising an apertured base plate (1) for securing the device to the skin surrounding the stoma, a ring (3; 13) connected with said base plate via a flexible connecting piece (2), and a bag (4) for taking up the excreta and being detachably attached to the ring, characterized in that the connecting piece (2) between the ring (3; 13) and the base plate (1)

(a) is cylindrical or conical,

(b) extends perpendicularly to the base plate (1) or obliquely downwards, and

(c) has a diameter which compared with the base plate (1) is as large as possible.

2. The device of claim 1, characterized in that the connecting piece (2) is at least 1 mm high.

3. The device of claim 2, characterized in that the connecting piece (2) is 3 to 15 mm high.

4. The device of claims 1 to 3, characterized in that the edge (11) between the base plate (1) and the connecting piece (2) is rounded off.

5. The device of claims 1 to 4, characterized in that the connecting piece (2) is welded or glued with the base plate (1).

6. The device of claims 1 to 5, characterized in that the ring is a flat, flexible but selfsustaining non-deformable ring (3) for adhering bags and the bag (4) is an adhesive bag.

7. The device of claims 1 to 6, characterized in that the ring is a snap ring (13) and the bag (4) exhibits the counterpart of a spring catch.

8. The device of claims 1 to 7, characterized in that the connecting piece (2) seals the ring (3; 13) in its whole inner circumference.

9. The device of claim 8, characterized in that the connecting piece (2) is in part or in its whole circumference extended beyond the ring (3; 13), as a soil repeller (10), optionally by changing its form.

10. The device of claim 9, characterized in that the soil repeller (10) is extended to a nonreturn valve (12).

11. The device of claims 1 to 10, characterized in that the inner side of the connecting piece (2) is covered with silicon.

12. The device of claims 1 to 11, characterized in that the base plate (1) consists of a film with an adhesive conventional in adhesive bags.

## Patentansprüche

1. Einrichtung zur Versorgung eines Stoma, umfassend eine mit einer Öffung versehene Basisplatte (1) zur Befestigung der Einrichtung an der das Stoma umgebenden Haut, einen mit der Basisplatte über ein biegsames Verbindungsstück (2) verbundenen Ring (3; 13), und einen Beutel (4) zur Aufnahme der Exkrete, welcher abnehmbar mit dem Ring verbunden ist, dadurch gekennzeichnet, daß das Verbindungsstück (2) zwischen dem Ring (3; 13) und der Basisplatte (1)

(a) zylindrisch oder konisch ist,

(b) sich senkrecht zu der Basisplatte (1) oder schräg nach unten erstreckt, und

(c) einen Durchmesser aufweist, der im Vergleich zur Basisplatte (1) so groß wie möglich ist.

2. Einrichtung nach Anspruch 1, dadurch gekennzeichnet, daß das Verbindungsstück (2) mindestens 1 mm hoch ist.

3. Einrichtung nach Anspruch 2, dadurch gekennzeichnet, daß das Verbindungsstück (2) 3 bis 15 mm hoch ist.

4. Einrichtung nach den Ansprüchen 1 bis 3, dadurch gekennzeichnet, daß die Kante (11) zwischen der Basisplatte (1) und dem Verbindungsstück (2) abgerundet ist.

5. Einrichtung nach den Ansprüchen 1 bis 4, dadurch gekennzeichnet, daß das Verbindungsstück (2) mit der Basisplatte (1) verschweißt oder verklebt ist.

6. Einrichtung nach den Ansprüchen 1 bis 5, dadurch gekennzeichnet, daß der Ring ein flacher, biegsamer, jedoch selbsttragender, nicht verformbarer Ring (3) zum Ankleben von Beuteln ist und daß der Beutel (4) ein Klebebeutel ist.

7. Einrichtung nach den Ansprüchen 1 bis 6, dadurch gekennzeichnet, daß der Ring ein Schnappring (13) ist, und der Beutel (4) das Gegenstück eines Schnappverschlusses darstellt.

8. Einrichtung nach den Ansprüchen 1 bis 7, dadurch gekennzeichnet, daß das Verbindungsstück (2) den Ring (3; 13) an seinem gesamten inneren Umfang abdichtet.

9. Einrichtung nach Anspruch 8, dadurch gekennzeichnet, daß das Verbindungsstück (2) zum Teil oder in seinem gesamten Umfang als Schmutzabweiser (10), gegebenenfalls unter Änderung seiner Form, über den Ring (3; 13) verlängert ist.

10. Einrichtung nach Anspruch 9, dadurch gekennzeichnet, daß der Schmutzabweiser (10) zu einem Rückschlagventil (12) verlängert ist.

11. Einrichtung nach Ansprüchen 1 bis 10, dadurch gekennzeichnet, daß die Innenseite des Verbindungsstückes (2) mit Silikon beschichtet ist.

12. Einrichtung nach den Ansprüchen 1 bis 11, dadurch gekennzeichnet, daß die Basisplatte (1) aus einer Folie mit einem bei Klebebeuteln üblichen Klebstoff besteht.

## Revendications

1. Dispositif permettant les soins d'une ouverture de stomie, comprenant une plaque de base (1) percée d'une ouverture pour fixer le dispositif à la peau qui entoure l'ouverture de stomie, un anneau (3; 13) raccordé à ladite plaque de base par l'intermédiaire d'une pièce de raccordement souple (2), et une poche (4) destinée à recueillir les excreta et fixée de manière amovible à l'anneau, caractérisé en ce que la pièce de raccordement (2) entre l'anneau (3; 13) et la plaque de base (1)

(a) est cylindrique ou conique,

(b) est dirigée perpendiculairement à la plaque de base (1) ou obliquement vers le bas, et

(c) a un diamètre qui, comparé à celui de la plaque de base (1), est aussi grand que possible.

2. Dispositif selon la revendication 1, caractérisé en ce que la pièce de raccordement (2) est haute d'au moins 1 mm.

3. Dispositif selon la revendication 2, caractérisé en ce que la pièce de raccordement (2) est haute de 3 à 15 mm.

4. Dispositif selon les revendications 1 à 3, caractérisé en ce que le bord (11) entre la plaque de base (1) et la pièce de raccordement (2) est arrondi.

5. Dispositif selon les revendications 1 à 4, caractérisé en ce que la pièce de raccordement (2) est soudée ou collée à la plaque de base (1).

6. Dispositif selon les revendications 1 à 5, caractérisé en ce que l'anneau est un anneau non déformable (3) plat et souple mais autoporteur permettant de coller les poches, et la poche (4) est une poche adhésive.

7. Dispositif selon les revendications 1 à 6, caractérisé en ce que l'anneau est une bague élastique (13) et la poche (4) porte la partie complémentaire d'un taquet à ressort.

8. Dispositif selon les revendications 1 à 7, caractérisé en ce que la pièce de raccordement (2) ferme hermétiquement l'anneau (3; 13) sur tout son pourtour interne.

9. Dispositif selon la revendication 8, caractérisé en ce que la pièce de raccordement (2) se prolonge, sur une partie ou sur la totalité de sa circonférence, au-delà de l'anneau (3; 13), en formant un dispositif antisalissures (10), facultativement en changeant de forme.

10. Dispositif selon la revendication 9, caractérisé en ce que le dispositif antisalissures (10) se prolonge jusqu'à un clapet antiretour (12).

11. Dispositif selon les revendications 1 à 10, caractérisé en ce que la face intérieure de la pièce de raccordement (2) est recouverte de silicone.

12. Dispositif selon les revendications 1 à 11, caractérisé en ce que la plaque de base (1) se compose d'une pellicule recouverte d'un adhésif classique pour les poches adhésives.

Fig.4a

Fig.4b

Fig. 5a

Fig. 5b

Fig.1

Fig. 2

Fig.3